# EUROPEAN PATENT SPECIFICATION

(11) **EP 2 415 437 B1**
(45) Date of publication and mention of the grant of the patent: **07.01.2015**
(21) Application number: 10758824.6
(22) Date of filing: 31.03.2010
(51) Int. Cl.: A61F 13/15, A61F 13/472, A61F 13/49, A61F 13/42, A61F 13/84, A61L 15/42

(54) **Absorbent article**
Saugfähiger Artikel
Article Absorbant

(30) Priority: 31.03.2009 JP 2009088487
(43) Date of publication of application: 08.02.2012
(73) Proprietor: Unicharm Corporation, Ehime 799-0111 (JP)
(72) Inventor: KOMATSU, Shinpei, Kanonji-shi Kagawa 769-1602 (JP); NODA, Yuki, Kanonji-shi Kagawa 769-1602 (JP)
(74) Representative: Eke, Philippa Dianne
(86) International application number: PCT/JP2010/055921
(87) International publication number: WO 2010/114053

(56) References cited:
- WO-A1-2008/117755
- GB-A- 2 390 546
- JP-A- 6 056 664
- JP-A- 2005 287 793
- JP-A- 2007 222 598
- JP-A- 2008 006 277
- JP-B2- 3 265 596
- JP-T- 2002 505 125
- JP-T- 2004 536 048
- JP-T- 2007 525 245
- US-A1- 2002 169 427
- US-A1- 2003 028 163
- US-A1- 2006 069 364

## Description

### Technical Field

The present invention relates to an absorbent article that includes a topsheet, a backsheet, and an absorber.

### Background Art

Conventionally, in an absorbent article such as a disposable diaper, a cooling sensation agent that imparts a cooling sensation for the wearer is sometimes disposed in the skin contact surface that is in contact with the wearer's skin (see Patent Document 1). The cooling sensation agent is enveloped by a micro-encapsulation agent that is dissolved by water, and then placed on the skin contact surface that is in contact with the wearer's skin. When the membrane is dissolved by the wearer's urine, the components of the cooling sensation agent are released, creating a stimulus by coming into contact with the wearer's skin. The wearer can thereby be aware of time to exchange the diaper. US patent application no. US 2002/0169427 (Patent Document 3) describes a wearing article having a temperature change element.

An absorbent article has also been disclosed in which a substance is applied to impart a refreshing sensation to the wearer, not only with the function of suggesting the "time to exchange" as described above but also with the objective of, for example, reducing the steaminess or stickiness when worn (see Patent Document 2).

However, as described above, the conventional absorbent article having a function of imparting cooling sensation to a wearer has entailed the following problem. A compound imparting cooling sensation (hereinafter, referred to as a refresher agent) is disposed at a position coming into direct contact with a wearer's skin; and therefore, some wearers feel that stimulation is too strong. Excessive stimulation gives the wearer a sense of discomfort rather than a refreshing sensation.

### [Related Art Document]

### [Patent Document]

Patent Document 1: JP-A-2008-006277 (Page 4, Fig. 1)
Patent Document 2: JP-A-2007-525245
Patent Document 3: US 2002/0169427

The present invention provides the absorbent article of independent Claim 1. The dependent claims specify preferred but optional features.
An absorbent article includes a topsheet having a contact surface that comes into contact with a skin of a wearer; a liquid-impermeable backsheet which does not permeate a liquid; and an absorber that is arranged between the topsheet and the backsheet., wherein a material including a refresher agent is provided between the topsheet and the backsheet, and
the material including the refresher agent is arranged at each side in a widthwise direction of the absorber.

### [Brief Description of Drawings]

[Fig. 1] Fig. 1 is a plan view of an absorbent article according to an embodiment.
[Fig. 2] Fig. 2 is a sectional view taken along the line F1-F1'shown in Fig. 1.
[Fig. 3] Fig. 3 is a structural view illustrating an outline of an apparatus for disposing a cool feeling material.
[Fig. 4] Fig. 4 is a view illustrating an outline of a coater applying apparatus.
[Fig. 5] Fig. 5 is a view showing an example of a coating pattern that can be implemented by employing the coater applying apparatus.
[Fig. 6] Fig. 6 is a view showing a coating processing pattern in which a cooling sensation agent is disposed at a discontinuous position in an MD direction.

### Best Mode for Carrying out the Invention

A description is provided for the absorbent article according to the embodiments, with reference to the drawings. In the following description of the drawings, identical or analogous parts are given identical or analogous reference numerals. It must be noted that the drawings are schematic ones, and the respective dimensional ratios and the like may differ from reality.

Accordingly the specific dimensions and the like must be determined by consulting the following description. It is a matter of course that the interrelationships of the drawings also contain parts with mutually differing dimensional ratios and relationships.

### (Structure of Absorbent Article)

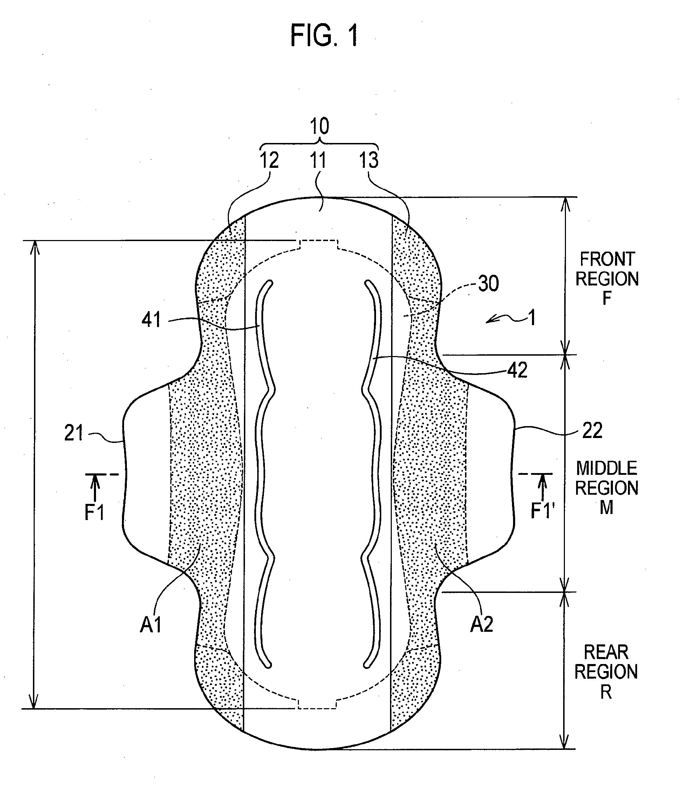

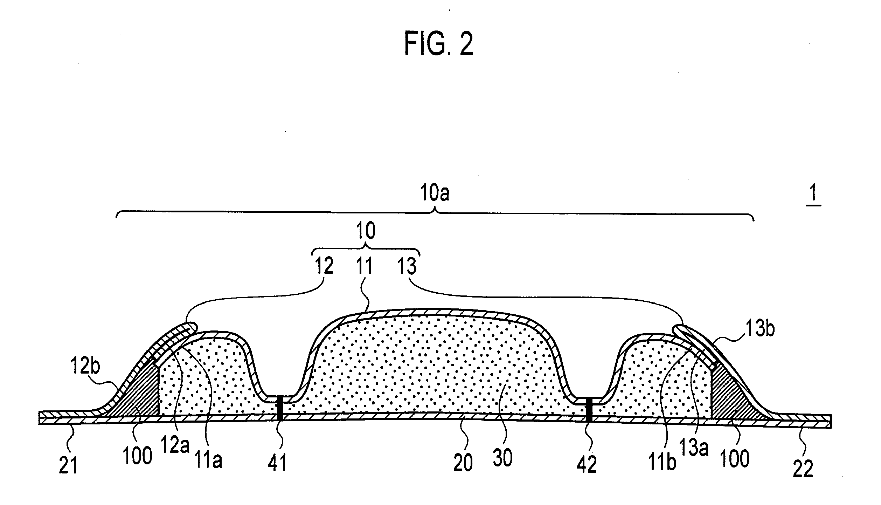

An absorbent article according to an embodiment will be described with reference to Fig. 1 and Fig. 2. Fig. 1 is a plan view of an absorbent article 1 according to the embodiment. Fig. 2 is a sectional view taken along the line F1-F1' shown in Fig. 1.

The absorbent article 1 in Fig. 1 is, for example, a sanitary napkin. As illustrated in Fig. 1, the absorbent article 1 has a front region F, a middle region M, and a rear region R. The front region F is in contact with the surface of the skin on the wearer's ventral side. The middle region M is in contact with the surface of the skin around the wearer's vaginal orifice. The rear region R is in contact with the surface of the skin on the wearer's buttocks side.

The absorbent article 1 has a topsheet 10 in contact with the wearer's skin, a liquid-impermeable backsheet 20 through which liquids cannot pass, and an absorber 30. The absorbent article 1 includes a material 100 that contains a compound for giving the wearer a refreshing sensation (hereinafter, called a refresher agent) between the topsheet 10 and the backsheet 20. In this embodiment, the material that contains the refresher agent is referred to as the cooling sensation material 100.

The absorber 30 is arranged between the topsheet 10 and the backsheet 20. Therefore, the absorber 30 is indicated by the dotted line in Fig. 1. The absorber 30 is arranged at a middle portion in a longitudinal direction of the absorbent article 1. The cool feeling material 100 is arranged at each side in a widthwise direction of the absorber 30. That is, the cooling sensation material 100 is arranged at a position corresponding to regions A1 and A2 indicated by shading and in a space between the topsheet 10 and the backsheet 20 in Fig. 1.

The topsheet 10 has: a first sheet 11 covering at least a surface of a wearer's side of the absorber 30; and second sheets 12 and 13 that are disposed at both sides in a widthwise direction of the first sheet 11.

The backsheet 20 has a wing 21 and a wing 22. The wing 21 and the wing 22 are formed in one pair at positions that correspond to a widthwise direction of the absorbent article 1. The wing 21 and the wing 22 are extended in the widthwise direction of the absorbent article 1 in the middle region M. A width of the middle region M of the backsheet 20 is greater than that of each of the forward region F and the rearward region R.

The first sheet 11 has a length which is substantially identical to that of the backsheet 20. A shape of an end part of the first sheet 11 is substantially identical to that of the backsheet 20. The first sheet 11 covers at least a surface of the absorber 30.

The second sheets 12 and 13 are arranged at both sides of the first sheet 11. The second sheet 12 covers a part of an end part of the absorber 30 and the wing 21. One end part in a longitudinal direction of the second sheet 12 is a substantially straight line, and is superimposed on one end part in a longitudinal direction of the first sheet 11. The other end part in the longitudinal direction of the second sheet 12 is coincident with a part of an outer circumference of the backsheet 20 and a shape of the wing 21.

It is preferable that dimensions in the longitudinal direction of the absorbent article 1 be within the range of 100 mm to 500 mm, and specifically it is further preferable that the dimensions be within the range of 150 mm to 350 mm. In addition, it is preferable that dimensions of the widthwise direction be within the range of 30 mm to 200 mm, and specifically it is further preferable that the dimensions be within the range of 40 mm to 180 mm.

The second sheet 13 covers a part of a side edge of the absorber 30 and the wing 22. One end part in a longitudinal direction of the second sheet 13 is a substantially straight line, and is superimposed on the other end part in the longitudinal direction of the first sheet 11. The other end part in the longitudinal direction of the second sheet 13 is coincident with a part of an outer circumference of the backsheet 20 and a shape of the wing 22.

Fig. 2 is a sectional view taken along the line F1-F1' of the absorbent article 1. As shown in Fig. 2, the absorber 30 is arranged between the topsheet 10 and the backsheet 20. The wing 21 is covered with the second sheet 12. The wing 22 is covered with the second sheet 13.

As shown in Fig. 2, the second sheets 12 and 13 are bonded with the first sheet 11 at edge parts 11a and 11b in the widthwise direction of the first sheet 11. The cooling sensation material 100 is arranged at a back face side of each of the second sheets 12 and 13. The cooling sensation material 100 comes into contact with the back faces of the second sheets 12 and 13.

In addition, the second sheet 12 has: an edge part 12a in a widthwise direction of the second sheet 12; and a fold part that is positioned inside in a widthwise direction more than the edge part 12a. The second sheet 12 is superimposed on the first sheet 11, and in a state in which the edge part 11a in the widthwise direction of the first sheet 11 and the edge part 12a of the second sheet 12 are bonded with each other, the fold part 12b is folded to the outside in the widthwise direction of the first sheet 11, whereby a contact surface 10a coming into contact with a wearer's skin is formed. Like the second sheet 12, the second sheet 13 also has: an edge part 13a in a widthwise direction of the second sheet 13; and a fold part 13b that is positioned inside in the widthwise direction more than the edge part 13a, and is bonded with the end part 11b of the first sheet.

In addition, in the absorbent article 1, the first sheet 11, the second sheets 12 and 13, the backsheet 20, and the absorber 30 are bonded with each other. Circumferential edges of the first sheet 11, the second sheets 12 and 13, and the backsheet 20 are bonded with each other, and the absorber 30 is internally sealed.

As a method of bonding the topsheet 10 and the backsheet 20 with each other, it is possible to employ one of heat emboss processing and ultrasonic or hot melt type bonding agent or a combination of a plurality thereof. The first sheet 11 and the absorber 30 are brought into pressure contact with each other by means of a pressure contact unit 41 and a pressure contact unit 42. The pressure contact unit 41 and the pressure contact unit 42 are formed along a longitudinal direction of the absorber 30 at both sides in the widthwise direction of the absorber 30. In the embodiment, the pressure contact unit 41 and the pressure contact unit 42 are brought into pressure contact with each other by means of heat emboss processing.

In the backsheet 20, on a surface coming into contact with shorts, an adhesive agent, although not shown, is applied in a linear shape, along the longitudinal direction of the backsheet 20. The adhesive agent is applied in a plurality of lines along the longitudinal direction of the backsheet 20. In the wing 21 and the wing 22, an adhesive agent is also applied to a surface coming into contact shorts. A protective sheet for retaining stickiness is adhered to an adhesive material. The protective sheet is released by a wearer at the time of use of the absorbent article.

Next, the first sheets 11 and 12 will be specifically described.

In the embodiment, the first sheet 11 is a nonwoven cloth. A material for the first sheet 11 is not limitative in particular as long as it is a sheet-like material with its structure of permeating a liquid, such as a porous plastic sheet. As a material for cloth or nonwoven cloth, either of a natural fiber and a chemical fiber can be used.

As examples of natural fibers, there are exemplified celluloses such as ground pulp or cotton. As examples of chemical fibers, there are exemplified: reproducible celluloses such as rayon or fibril rayon; semisynthetic celluloses such as acetate or triacetate; thermoplastic hydrophobic chemical fibers; or thermoplastic hydrophobic chemical fibers or the like to which hydrophilic treatment is applied.

Examples of thermoplastic hydrophobic chemical fibers, there are exemplified a single fiber such as polyethylene (PE), polypropylene (PP), or polyethylene terephthalate (PET); a fiber made by graft-polymerization of polyethylene and polypropylene; or a composite fiber of a core-clad structure or the like.

As a method of web forming of a nonwoven cloth, there can be employed any one method of a dry type (a card technique, a spun bond technique, a melt blow technique, or an air-laid technique) and a wet type. Among the dry type technique and the wet type technique, a plurality of techniques may be combined with each other. In addition, a method such as thermal bonding, needle punching, or chemical bonding is exemplified. A method of producing a nonwoven cloth is not limitative to the methods described above.

Further, a spun lace formed in a sheet shape by means of a water entangling technique can be employed for the first sheet 11. Furthermore, a nonwoven cloth obtained by applying irregularities to a top layer side of the unwoven cloth or an irregular nonwoven cloth at which a total weight non-uniformity is provided by applying air at the time of forming webs can be employed for the first sheet 11. By forming irregularities on a surface, it is possible to alleviate a bodily liquid from diffusing along a surface of the first sheet 11 before permeating the first sheet 11.

The second sheets 12 and 13 can be selected from among the materials similar to those for the first sheet 11. However, it is preferable to have hydrophobic property or water repellent property in order to prevent menstrual blood from flowing over the second sheets 12 and 13 outward of the absorbent article 1. Specifically a spun bond nonwoven cloth, an SMS nonwoven cloth or the like is exemplified. The second sheets 12 and 13 constitute a skin contact surface 10a. Therefore, it is preferable to employ an air-through nonwoven cloth in order to reduce frictional stimulation to skin.

Next, the backsheet 20 will be specifically described.

In the embodiment, as the backsheet 20, there can be employed: a film which consists essentially of polyethylene, polypropylene or the like; an air ventilation resin film; or a sheet or the like obtained by bonding a ventilation resin film with a nonwoven cloth such as a spun bond or a spun lace. It is preferable that the backsheet 20 be a material having its flexibility to an extent such that a sense of discomfort at the time of wearing is unlikely to occur. For example, it is preferable to use a film which consists essentially of a low-density polyethylene (LDPE) resin, a total weight (weight (g) per unit area) of which is within the range of 15 g/m² to 30 g/m².

Next, the absorber 30 will be specifically described.

The absorber 30 includes a hydrophilic fiber or pulp. As examples of hydrophilic fibers, there can be employed solely or in combination: celluloses such as ground pulp or cotton; reproducible celluloses such as rayon or fibril rayon; semisynthetic celluloses such as acetate or triacetate; particle-like polymers, fiber-like polymers, thermoplastic hydrophobic chemical fibers, or thermoplastic hydrophobic chemical fibers to which hydrophilic treatment is applied. Among them, it is preferable to use crushed pulp in consideration of low cost and easiness of molding an absorber.

The absorber 30 may be constituted by covering a hydrophilic fiber and a polymeric absorber with a coating material. In the embodiment, the polymeric absorber is a grain-like polymer such as sodium acrylate copolymer having absorbability and hygroscopic property. In addition, to the absorber 30, there may be added, for example, grain-like deodorant materials such as silver, copper, zinc, silica, activated carbon, aluminosilicate compound, or zeolite, copper, zinc, silica, activated carbon, aluminosilicate, or zeolite or grain-like antimicrobial. Further, a grain-like refresher agent having its cooling effect due to heat absorption reaction may be added thereto.

The absorber 30 may be an air-laid sheet obtained by molding a hydrophilic fiber or powder in a sheet shape by means of an air-laid technique. In a case where the air-laid sheet is used as the absorber 30, it is preferable that a thickness of the air-laid sheet be 0.3 mm to 5.0 mm. As an example of air-laid sheet, there is employed the one obtained by molding a fiber and a grain-like polymer to be a sheet material by means of a binder or the like. The grain-like polymer may be dispersed in a layered shape or may be biased in a thickness direction in the air-laid sheet.

On the absorber 30, embosses may be formed in order to prevent deformation or folding during wearing or to adjust a thickness. These embosses for the absorber 30 can be formed by passing the absorber between a patterned emboss roll and a flat roll. While the pattern for the emboss roll is formed in a lattice shape, in a dotted shape, or in a wavy shape, it is preferable to employ a lattice-shaped pattern which is easily adjusted in thickness.

Next, the cooling sensation material 100 will be specifically described.

In the embodiment, it is preferable that a cooling sensation material 100 be carried by a porous material which is capable of adsorbing molecules by a plenty of small pores. The refresher agent is menthol, camphor, and thiol or the like, which are cyclo-hexanol derivatives, for example. In addition, the porous material is silica gel, alumina, zeolite, a nano-porous material or the like, for example.

The refresher agent carried (adsorbed) by the porous material is substituted by water (water steam) by means of capillary coagulation under a high humidity, and is discharged to the outside of the porous material. An interrelationship between a small pore diameter and a relative humidity of the porous material with which capillary coagulation occurs is in accordance with a so called Kelvin formula.

A humidity that is generally determined to be "confortable" is said to be 45% to 55%. In addition, when a temperature in shorts is 33 degrees C to 37 degrees C, if humidity reaches the order of 60%, a result that a wearer feels heating is obtained.

Therefore, a porous material is used which has a small pore diameter in which there occurs substitution between molecules and water content in which the small pore diameter of the porous material is adsorbed under a desired humidity. Specifically, the porous material has a small pore diameter in which water steam causes a capillary coagulation when the temperature is within the range of 20 degrees C to 40 degrees C and the relative humidity is within the range of 60% to 85%. In addition, small porous each having its small porous diameter that meets a condition in which water steam causes a capillary coagulation when the temperature is within the range of 20 degrees C to 40 degrees C and the relative humidity is within the range of 60% to 85% occupy 30% to 100% of all of the small pores of the porous material.

As one example, in a case where the porous material is silica gel, the small pore diameter is within the range of 4 nm to 13 nm according to the Kelvin formula. If the small pore diameter is smaller than 4 nm, the refresher agent is discharged in a state in which the relative humidity is lower than 60%, that is, under a confortable condition in which a wearer does not feel heating.

Alternatively in a case where the small pore diameter exceeds 13 nm, since the refresher agent is not discharged even if the humidity exceeds 85%, a wearer is disallowed to have cooling sensation even under a condition in which the wearer feels heating. In addition, it is preferable that an average particle size of the porous material be 1.5 mm or less. If the average particle size of the porous material exceeds 1.5 mm, a wearer feels a sense of discomfort.

While the amount of the refresher agent to be carried by the porous material is different depending on the average particle size, small pore diameter distributions or the like, of the porous material, it is preferable that the amount be within the range of 1 g/m² to 100 g/m². If the amount is 1g/m² or less, it is difficult to impart cooling sensation to a wearer. Alternatively, if 100 g/m² is exceeded, it is not preferable, since stimulation is too strong for the wearer.

As described above, the refresher agent is disposed at a predetermined position in a state in which the agent is carried by the porous material. In addition, a refresher agent such as menthyl lactate or menthone glycerin acetal, which is soluble in a specific solvent, can be applied at a predetermined position in a state of solution.

### (Method 1 of Disposing Cool Sensation Material)

There are several disposition methods of disposing the cooling sensation material 100 at the position illustrated in the abovementioned embodiment, as set forth below. The disposition method 1 is a method of disposing the cooling sensation material 100 in a case where a refresher agent is carried by a porous material. Specifically in a region in which a hot melt type adhesive agent is applied, the cooling sensation material 100 is adhered by employing a roll.

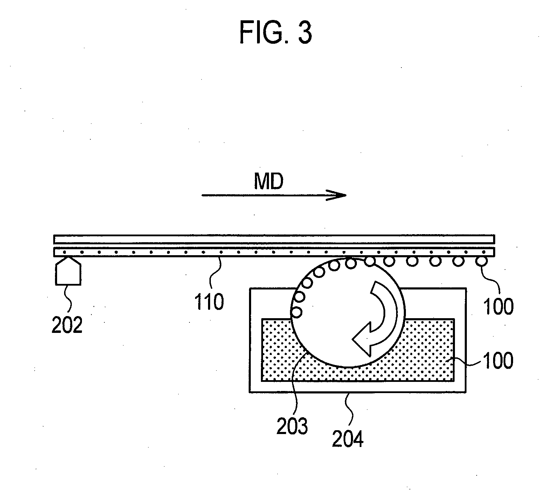

Fig. 3 is a structural view illustrating an outline of an arrangement device 200 for disposing the cooling sensation material 100. As shown in Fig. 3, the arrangement device 200 has: a conveyance unit 201 configured to convey a material 110 in a predetermined conveyance direction (an MD direction); and an HMA coating unit 202 configured to apply a hot melt type adhesive agent (hereinafter, referred to as HMA) onto a surface of the material 110. In addition, this arrangement device 200 has: a surface treatment roller 203 configured to dispose the cooling sensation material 100 onto the surface of the material 110 to which HMA is applied; and a reservoir 204 configured to contain the cooling sensation material 100. On the surface treatment roller 203, small pores are formed on a surface of the roll.

If the surface treatment roller 203 rotates in a state in which the roll is brought into contact with the cooling sensation material 100 that is reserved in the reservoir 204, the small pores formed on the surface of the roll are charged with the cooling sensation material 100. The surface treatment roller 203 comes into contact with the surface of the material 110, whereby the cooling sensation material 100 is transferred to the surface of the material 110 to which HMA is applied.

Here, the material 110 is a respective one of the second sheets 12 and 13 or the backsheet 20, for example. That is, by means of the arrangement device 200 of Fig. 3, the cooling sensation material 100 is disposed on a back face of each of the second sheets 12 and 13 (an opposite surface of a skin contact surface coming into contact with a wearer's skin) or at a skin side of the backsheet 20. Since the cooling sensation material 100 is adhered to the surface of the material 110 by means of HMA, the amount of the cooling sensation material 100 to be disposed per unit area in the material 110 can be varied by varying applying intervals of HMA or an applying area or the like. It is preferable that the applying quantity of HMA be within the range of 5 g/m² to 100 g/m². In addition, it is preferable that the applying intervals of HMA be 0.5 mm in width of the MD direction, and that the intervals in the MD direction be 0.5 mm or more.

In addition, since, in this disposition method, the cooling sensation material 100 is adhered to the second sheets 12 and 13 or the backsheet 20 by means of HMA, it is possible to prevent the cooling sensation material 100 from slipping off from a predetermined disposition position (regions A1 and A2), and it is also possible for a wearer to have a comfortable cooling sensation.

In a case where a heat vulnerable material is used as the backsheet 20, it may also be difficult to apply HMA to the backsheet 20. Therefore, it is preferable to dispose such material on a back face of each of the second sheets 12 and 13.

### (Second Method of Arranging Cooling Sensation Agent)

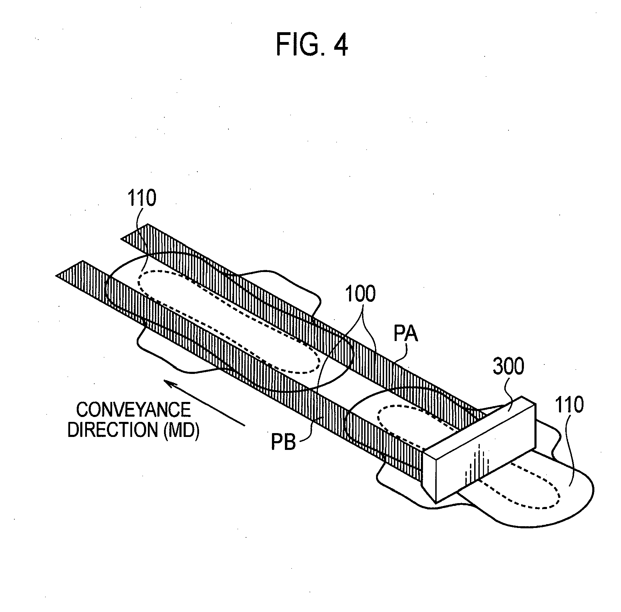

The second arrangement method is a method in which a refresher agent, such as methyl lactate and menthone glycerin acetal, soluble in a specific solvent, is applied to a given position while being a solution. When the refresher agent is a solution, a coater application can be used. Fig. 4 is a diagram illustrating the schematics of a coater application device.

The cooling sensation material 100 is applied to a surface in a predetermined region of the material 110 by means of a coater 300. The cooling sensation material 100 is applied to applying regions PA and PB. This cool feeling material is conveyed while a longitudinal direction of the material 110 that constitutes an absorbent article is made coincident with the conveyance direction MD.

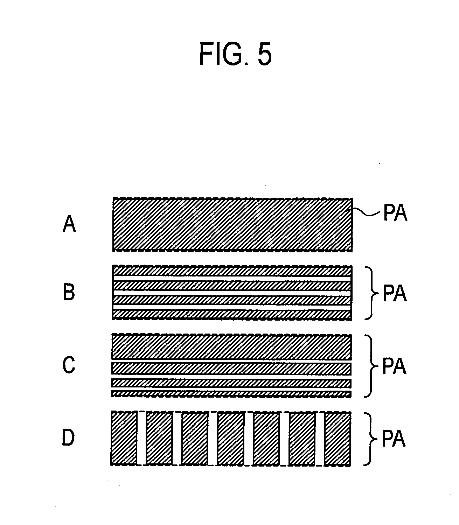

Fig. 5 shows examples of applying patterns of the cooling sensation material 100 to be disposed in the applying region PA, which can be implemented in a case of employing a coater applying processing apparatus. There are exemplified: a pattern A in which the cooling sensation material 100 is to be applied to a full face of the applying region PA; a pattern B in which the cooling sensation material 100 is to be applied in a striped manner to an applying region; a pattern C in which the cooling sensation material 100 is to be applied so as to be widened to the outside in a widthwise direction of the absorbent article, in Fig. 4; and a pattern D or the like in which the cooling sensation material 100 is intermittently applied in a flow direction. The applying quantity of the cooling sensation material 100 is preferably within the range of 0.5 g/m² to 5 g/m², in a case of employing L-menthol-containing Microshere (available from Symrise Inc.), a menthol rate of which is 25%, for example.

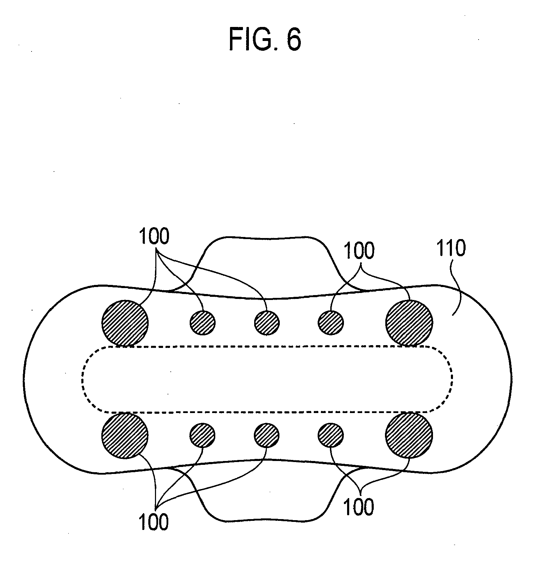

Further, by employing a so called flexographic coating method or gravure coating method or the like, as shown in Fig. 6, the cooling sensation material 100 can be disposed at a discontinuous position in the MD direction.

As described above, according to the absorbent article 1 according to the embodiment, the cooling sensation material 100 is arranged between the topsheet 10 and the backsheet 20 and at each side in the widthwise direction of the absorber 30, whereby the cooling sensation agent 100 does not come into direct contact with a wearer's skin.

The cooling sensation material 100 is discharged from a porous material under a high humidity environment. The discharged cooling sensation material 100 comes into contact with a skin cool feeling acceptance device TRPM8 (CMR1), and increases a threshold value of the acceptance device in order of 0 degrees C to 5 degrees C. In this manner, an effective temperature of a skin is reduced in order of 0 degrees C to 5 degrees C in a state in which a skin environment temperature is kept unchanged. Therefore, a wearer can obtain cooling sensation, since the effective temperature is reduced under an environment in which the wearer feels hot.

Further, the cooling sensation material 100 is disposed at a position which does not come into direct contact with a bodily liquid (water content). Therefore, it is possible to prevent discharge of a cooling material due to the cooling sensation material 100 coming into direct contact with the bodily liquid. In this manner, a cooling action can be sustained. In addition, before a condition in which a wearer feels hot is established, the cool feeling material comes into contact with water content to be thereby able to prevent a malfunction that a cooling material is discharged.

The cooling sensation material 100 is arranged at a back face side of each of the second sheets 12 and 13. In a case where the second sheets 12 and 13 are liquid-impermeable, an advantageous effect that the cooling sensation material 100 does not come into direct contact with water content can be enhanced. Therefore, a cooling material is not discharged until a condition in which a wearer feels hot is established.

In addition, in the absorbent article 1, the second sheet 12 is superimposed on the first sheet 11, and in a state in which the edge part 11a in the widthwise direction of the first sheet 11 and the edge part 12a of the second sheet 12 are bonded with each other, the fold part 12b is folded back to the outside in the widthwise direction of the first sheet 11, whereby the contact surface 10a coming into contact with a wearer's skin is formed. In this manner, it is possible to prevent water from leaking out from between the adhered sheets to the back face side of each of the second sheets 12 and 13, in comparison with a case of adhering back faces of the first sheet 11 and the second sheets 12 and 13. Therefore, an advantageous effect of preventing the water content on the surface of the first sheet 11 from coming into contact with the cooling sensation material 100 is enhanced.

As described above, while the contents of the present invention were disclosed through the embodiment, it should not be understood that the discussion and drawings forming a part of this disclosure limit the present invention. From this disclosure, a variety of alternative embodiments, examples, and operational techniques would have been self-evident to one skilled in the art.

For example, the embodiment can be modified as follows. While the foregoing embodiments each described that an absorbent article is a sanitary napkin, the embodiment can be applied to a so called liner, an incontinence article or the like (referred to as an incontinence pad) as well.

In addition, the absorbent article is not limitative to a planar shape disclosed in Fig. 1 described above. The absorbent article may be formed in a shape conforming to the shape of a wearer's crotch and the shape of shorts. The planar shape of the absorbent article can be formed in a variety of shapes such as a rectangular shape, an elliptical shape, or a gourd shape.

In the absorbent article, a gather made of an elastic material such as a resilient material may be provided at each end part in the widthwise direction of the absorber in order to prevent side leakage of bodily liquid such as menstrual blood.

As a polymeric absorber, there can be employed a grain-like polymer such as a sodium acrylate copolymer having absorbability or absorbance. In addition, between the topsheet and the backsheet, apart from an absorber, a polymeric absorber, and a hygroscopic material, there may be arranged grain-like deodorants such as silver, copper, zinc, silica, activated carbon, aluminosilicate compound, or zeolite.

Some electrolytes that can be utilized as the hygroscopic material such as silver, copper, zinc, silica, activated carbon, aluminosilicate compound, and zeolite include those having efficacy in suppressing microbial breeding (an antibacterial effect or bactericidal project). For example, when an electrolyte having efficacy in suppressing microbial breeding is used as the hygroscopic material, then the efficacy in suppressing microbial breeding can be imparted to the absorbent article.

As described above, of course, the present invention includes a variety of embodiments or the like which are not described herein. Therefore, a technical scope of the present invention is defined by only specific matters of the invention according to the claims that are reasonable from the foregoing description.

### [Example]

A volunteer was instructed to wear a test product, and "hotness" was measured by means of functional evaluation.

### o Product shape

- Product length: 230 mm and product width: 100 mm
- Porous material: B-type silica gel (L-menthol 10%)
- Proportion: B-type silica gel 90% and L-menthol 10%
- Average particle size: 50 microns and average small pore diameter: 7.5 nm
- Time required for carrying treatment of drying silica gel after impregnated in menthol solution is within the range of 2 hours to 8 hours.
- Absorber: A mixture of pulp of 150 g/m² and absorptive polymer of 15 g/m². This mixture was pinched by means of a tissue of 15 g/m².
- Topsheet: PE/PET air-through 25 g/m²
- Second sheet: PE/PET air-through 20 g/m²
- Side sheet (second sheet): Polypropylene spun bond (hydrophobic) 20 g/m²
- Backsheet: Polyethylene film 25 g/m²

A tissue was employed as a covering material. L-menthol carrying B-type silica gel was disposed between the tissue and the mixture as an absorber, and then, was fixed by means of a hot melt adhesive agent. The resultant L-menthol carrying B-type silica gel was disposed between the second sheets 12 and 13 and the first sheet 11, all of which were fixedly bonded with a hot melt adhesive material. The disposition quantity was 50 g/m².

### o Testing

- Testing environment: temperature 27 degrees C and humidity 70%
- Clothing: Underwear, polo shirt, and skirt
A sample product was worn on shorts.

A small-sized temperature and humidity meter was mounted at a relative position to the perineum part on a sanitary napkin.

In the abovementioned environment, a volunteer was caused to maintain a standing posture for 60 minutes. As a result, the volunteer did not feel hot immediately after wearing (humidity 55%). Fifteen minutes after wearing, the volunteer felt cooling sensation after the humidity in the crotch had exceeded 60%. Thereafter, while the humidity rose up to 80% within 45 minutes, a result that no hotness was felt was obtained. This result is deemed to be because a cooling material was discharged under the testing condition.

### Industrial Applicability

According to the present invention, it is possible to prevent an excessive stimulation from being imparted to a wearer by means of a refresher agent, and it is also possible to reliably restrain a sense of discomfort due to hotness, stickiness or the like.

## Claims

1. An absorbent article (1) comprising: a topsheet (10) having a contact surface that comes into contact with a skin of a wearer; a liquid-impermeable backsheet (20) which does not permeate a liquid; and an absorber (30) that is arranged between the topsheet and the backsheet, wherein
a material (100) including a refresher agent is provided between the topsheet and the backsheet,
the material including the refresher agent is arranged at each side in a widthwise direction of the absorber,
and
wherein the topsheet has: a first sheet (11) configured to cover at least a surface of the wearer's side of the absorber; and a second sheet (12, 13) that is disposed at each side of a widthwise direction of the first sheet,
the second sheet is bonded with the first sheet at an edge part (11a) in the widthwise direction of the first sheet,
and
the material including the refresher agent is arranged at a back face side of the second sheet,
wherein the material including the refresher agent is adhesively bonded with a back face of the second sheet in a state in which the material is in contact with the back face of the second sheet, and
wherein the refresher agent is carried by a porous material.

2. The absorbent article according to claim 1, wherein the second sheet has: an edge part (12a, 13a) that is positioned at an outside in a widthwise direction of the second sheet; and
a fold part (12b, 13b) that is positioned at an inside in the widthwise direction of the second sheet more than the edge part, the second sheet is superimposed on the first sheet, and in a state in which an edge part in a widthwise direction of the first sheet and an edge part that is positioned to the outside in the widthwise direction of the second sheet are bonded with each other, the fold part is folded to the outside in the widthwise direction of the first sheet, thereby forming a contact surface (10a) that comes into contact with the skin of the wearer.

3. The absorbent article according to claim 1, wherein the first sheet is a liquid-permeable sheet configured to permeate a liquid, and
the second sheet is a liquid-impermeable sheet which does not permeate a liquid.

## Patentansprüche

1. Saugfähiger Artikel (1), der Folgendes umfasst:
eine oberste Schicht (10), die eine Kontaktfläche hat, die mit der Haut eines Trägers in Berührung kommt,
eine flüssigkeitsundurchlässige hintere Schicht (20), die keine Flüssigkeit durchlässt,
und einen Absorber (30), der zwischen der obersten Schicht und der hinteren Schicht angeordnet ist, wobei
ein Material (100), das ein Erfrischungsmittel beinhaltet, das zwischen der obersten Schicht und der hinteren Schicht bereitgestellt wird,
das Material, das das Erfrischungsmittel beinhaltet, auf jeder Seite in Breitenrichtung des Absorbers angeordnet ist und
wobei die oberste Schicht eine erste Schicht (11) hat, die so konfiguriert ist, dass sie wenigstens eine Fläche auf der Seite des Trägers des Absorbers deckt,
und eine zweite Schicht (12, 13), die auf jeder Seite in Breitenrichtung der ersten Schicht angeordnet ist, wobei die zweite Schicht mit der ersten Schicht an einem Randteil (11a) in Breitenrichtung der ersten Schicht angeordnet ist, und
das Material, einschließlich des Erfrischungsmittels, auf einer Seite der hinteren Fläche der zweiten Schicht angeordnet ist,
wobei das Material, einschließlich des Erfrischungsmittels, an einer hinteren Fläche des zweiten Schicht in einem Zustand haftend verbunden ist, in dem das Material mit der hinteren Fläche der zweiten Schicht in Berührung ist, und
wobei das Erfrischungsmittel von einem porösen Material getragen wird.

2. Absorbierender Artikel nach Anspruch 1, wobei die zweite Schicht Folgendes umfasst:
ein Randteil (12a, 13a), das auf einer Außenseite in Breitenrichtung der zweiten Schicht positioniert ist, und
ein gefaltetes Teil (12b, 13b), das auf einer Innenseite in Breitenrichtung der zweiten Schicht positioniert ist, und mehr als der Randteil ist, und die zweite Schicht auf die erste Schicht gelegt wird, und in einem Zustand, in dem ein Randteil in Breitenrichtung der ersten Schicht ist, und ein Randteil, das an der Außenseite in Breitenrichtung der zweiten Schicht positioniert ist und aneinander haften, wobei das gefaltete Teil an der Außenseite in Breitenrichtung der ersten Schicht gefaltet ist, und somit eine Kontaktfläche (10a) formt, die mit der Haut des Trägers in Berührung kommt.

3. Saugfähiger Artikel nach Anspruch 1, wobei die erste Schicht eine flüssigkeitsdurchlässige Schicht ist, die so konfiguriert ist, dass sie eine Flüssigkeit durchlässt, und
die zweite Schicht eine flüssigkeitsundurchlässige Schicht ist, die keine Flüssigkeit durchlässt.

## Revendications

1. Article absorbant (1) comportant : une feuille de dessus (10) ayant une surface de contact qui entre en contact avec une peau d'un utilisateur ; une feuille de support imperméable aux liquides (20) qui ne laisse pas passer un liquide ; et un élément absorbant (30) qui est agencé entre la feuille de dessus et la feuille de support, dans lequel
un matériau (100) comprenant un agent rafraîchissant est mis en oeuvre entre la feuille de dessus et la feuille de support,
le matériau comprenant l'agent rafraîchissant est agencé au niveau de chaque côté dans une direction allant dans le sens de la largeur de l'élément absorbant, et
dans lequel la feuille de dessus a : une première feuille (11) configurée afin de recouvrir au moins une surface du côté utilisateur de l'élément absorbant ; et une deuxième feuille (12, 13) qui est disposée au niveau de chaque côté dans une direction allant dans le sens de la largeur de la première feuille,
la deuxième feuille est reliée à la première feuille au niveau d'une partie de bord (11a) dans la direction allant dans le sens de la largeur de la première feuille, et
le matériau comprenant l'agent rafraîchissant est agencé au niveau d'un côté de face arrière de la deuxième feuille,
dans lequel le matériau comprenant l'agent rafraîchissant est relié de manière adhésive à une face arrière de la deuxième feuille dans un état dans lequel le matériau est en contact avec la face arrière de la deuxième feuille, et
dans lequel l'agent rafraîchissant est porté par un matériau poreux.

2. Article absorbant selon la revendication 1, dans lequel la deuxième feuille a : une partie de bord (12a, 13a) qui est positionnée au niveau d'une partie extérieure dans une direction allant dans le sens de la largeur de la deuxième feuille ; et
une partie de pli (12b, 13b) qui est positionnée au niveau d'une partie intérieure dans la direction allant dans le sens de la largeur de la deuxième feuille plus que la partie de bord, la deuxième feuille est superposée sur la première feuille, et dans un état dans lequel une partie de bord dans une direction allant dans le sens de la largeur de la première feuille et une partie de bord qui est positionnée au niveau de la partie extérieure dans la direction allant dans le sens de la largeur de la deuxième feuille sont reliées l'une par rapport à l'autre, la partie de pli est pliée au niveau de la partie extérieure dans la direction allant dans le sens de la largeur de la première feuille, pour ainsi former une surface de contact (10a) qui entre en contact avec la peau de l'utilisateur.

3. Article absorbant selon la revendication 1, dans lequel la première feuille est une feuille perméable aux liquides configurée pour laisser passer un liquide, et
la deuxième feuille est une feuille imperméable aux liquides qui ne laisse pas passer un liquide.
